(19) Europäisches Patentamt
European Patent Office
Office européen des brevets

(11) EP 0 730 631 B1

(12) EUROPEAN PATENT SPECIFICATION

(45) Date of publication and mention of the grant of the patent:
**07.07.1999 Bulletin 1999/27**

(21) Application number: **95901547.0**

(22) Date of filing: **25.11.1994**

(51) Int Cl.[6]: **C11D 3/39**, C07D 227/087

(86) International application number:
**PCT/GB94/02583**

(87) International publication number:
**WO 95/14759 (01.06.1995 Gazette 1995/23)**

# (54) BLEACHING COMPOSITIONS

BLEICHMITTELZUSAMMENSETZUNGEN

COMPOSITIONS DE BLANCHIMENT

(84) Designated Contracting States:
**DE ES FR GB IT**

(30) Priority:
**25.11.1993 GB 9324261**
**16.12.1993 GB 9325777**
**23.12.1993 GB 9326382**
**23.12.1993 GB 9326385**
**23.12.1993 GB 9326383**
**23.12.1993 GB 9326384**

(43) Date of publication of application:
**11.09.1996 Bulletin 1996/37**

(73) Proprietor: **WARWICK INTERNATIONAL GROUP LIMITED (Company No. 2982784)**
**Holywell, Flintshire CH8 9HE (GB)**

(72) Inventors:
- **CROWTHER, Jan Darrel, Vaughn Close**
  **Clwyd LL19 8SH (GB)**
- **HAQUE, Ekram**
  **Deeside Clwyd CH5 1BL (GB)**
- **COLES, Barrie**
  **Clwyd CH8 8PN (GB)**

(74) Representative:
**Jones, Helen Marjorie Meredith et al**
**Gill Jennings & Every,**
**Broadgate House,**
**7 Eldon Street**
**London EC2M 7LH (GB)**

(56) References cited:

| | |
|---|---|
| EP-A- 0 122 763 | EP-A- 0 268 218 |
| EP-A- 0 400 971 | EP-A- 0 412 058 |
| WO-A-87/04429 | WO-A-94/27970 |
| WO-A-94/28102 | WO-A-95/00626 |
| WO-A-95/14075 | DE-A- 2 616 993 |
| GB-A- 1 596 313 | US-A- 4 113 735 |

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 730 631 B1

## Description

This invention relates to the in situ production of peroxygen-based oxidising species from a peroxygen source and an activator followed by the use of the product as an oxidising agent, for instance in a detergent or as a bleach or a biocide.

It is known that percarboxylic acid compounds are useful as bleaching agents, general oxidising agents and biocides. Such compounds may be incorporated into for instance laundry detergents, hard surface cleaners, paper and pulp bleaching compositions, fabric and fibre bleaching compositions and even depilatory creams as well as biocidal compositions, for use in agriculture.

It is also known to use a combination of peroxygen bleach precursor (or peroxygen source) and bleach activator in the same or separate compositions to form the oxidising species. The bleach activators are acyl-releasing agents. Suitable bleach activators are O-acyl or N-acyl compounds.

However, there is still a need for further bleach activators which will provide good bleaching and may also provide improved cleaning in detergent compositions.

In GB 855735 bleaching processes and compositions for textile material are described in which the bleaching composition is an aqueous solution comprising hydrogen peroxide and a reactive acyl organo-amide. A large range of compounds is included in this definition, including N-acyl lactams, of which the only exemplified lactam is N-acetyl caprolactam.

GB 1596313 discloses detergents for laundering textiles in which the compositions contain bleaching agents and an activator. The only activator mentioned is N-acetyl caprolactam. N-acetyl caprolactam is also mentioned in GB 2189520 A which relates to washing and bleaching processes. A typical example of a bleach precursor is given as N-acetyl caprolactam. Caprolactam in its N-acetylated form is also known as an ingredient in detergent compositions from SU 1664826.

In EP-A-0122763, bleach compositions are described in which an activator is adsorbed onto sodium perborate monohydrate. The activators claimed are any O-acyl or N-acyl activator and a large range of suitable compounds are disclosed. Within these compounds, N-acyl caprolactam is mentioned, where the acyl group is $R^aCO$ in which $R^a$ represents hydrogen or an alkyl group of chain length $C_1$ to $C_9$ aliphatic or aromatic group, optionally substituted by an alkyl or carboxylic acid group or the acyl is a di-acyl derived from a dicarboxylic acid having from 4 to 12 carbon atoms.

WO-A-95/00626 (prior art under Article 54 (3) EPC only) discloses valerolactam compounds for use as bleach activators, substantially corresponding to compounds of the formula I herein, in which R is a $C_4$ alkylene group.

WO-A-94/28102 and WO-A-94/27970 (prior art under Article 54(3) EPC only) describes caprolactam compounds for use as bleach activators. Specifically exemplified compounds mentioned in these specifications are not covered by the claims of the present case.

WO-A-95/14075 (prior art under Article 54 (3) EPC only) describes pyrrolidone compounds for use as bleach activators. The compounds have a $COOR^x$ substituent on the carbon atom joined to the pyrrolidone ring nitrogen atom. Such compounds are disclaimed herein.

The present invention relates to novel bleach compositions containing activators which provide improved cleaning properties.

In accordance with the present invention there is provided a composition containing a peroxygen source and a bleach activator comprising an optionally substituted N-acyl heterocycle having the formula I:

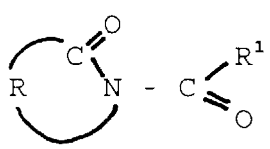

(I)

in which R represents an optionally substituted $C_3$, $C_5$ or $C_6$-alkylene function which forms a cyclic structure with - N- and -C(O)- and,

where R represents a $C_3$ or $C_6$ alkylene, $R^1$ represents a $C_{1-30}$ alkyl or aryl group; or has the formula $-R^2-COX$ where X may be OH, OM (where M is a metal ion), $-ON^+R^3_3$ (where $R^3$ are each independently selected from H and $C_{1-4}$ alkyl), a halogen atom, amine, $C_{1-30}$ alkyl or alkoxy or a leaving group and $R^2$ is a $C_{1-30}$ alkylene or arylene group disclaiming compounds in which R is $C_3$-alkylene which has a $COOR^x$ substituent at the carbon atom joined to the ring nitrogen atom, where $R^x$ is hydrogen $C_{1-4}$-alkyl, alkali metal or ammonium;

where R represents $C_5$ alkylene, it is $(CH_2)_5$ and $R^1$ is selected from 3,4-dicarboxyphenylanhydride, 5-(N-phthalimidoyl) pentyl, phenoxymethyl and tertiary butyl.

Thus, the compounds of the formulae I may be optionally substituted

N-acyl 5-membered heterocycles, N-acyl pyrrolidones having the formula (III):

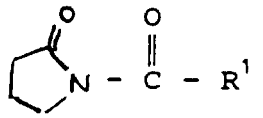

(III)

N-acyl 7-membered rings caprolactams of the formula (V):

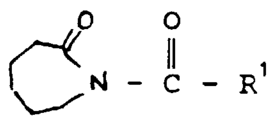

(V)

N-acyl 8-membered heterocycles (2-azocyclooctanones) of the formula (VI):

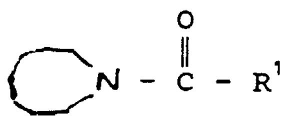

(VI)

In each of the formulae listed above, $R^1$ is as defined for formula I above.

Where the bleach activator is a 7-membered ring, R is $(CH_2)_5$ and $R^1$ is selected from 3,4-dicarboxyphenylanhydride, 5-(N-phthalimidoyl) pentyl, phenoxymethyl and tertiary butyl. Where the bleach activator is other than a 7-membered ring, preferably $R^1$ is a $C_{2-14}$, most preferably $C_{7-12}$ group. Particularly preferred groups are aryl especially phenyl, which are optionally substituted, or alkyl groups, preferably straight chain, especially ethyl. Although it is not preferred, $R^1$ may be a group linked to a substituent on the ring formed from -R- , -N- and -C(O)-, to form a cyclic structure.

Particularly preferred activators of the present invention for compounds of formula (III), (V) or (VI) are benzoyl N-substituted heterocycles.

In the present invention, unless specified, the term alkyl(ene) includes alkynyl(ene) and alkenyl(ene) groups and these groups may be straight chain, branched or may include cycloalkyl, groups. The alkyl groups may include heteroatoms such as N or O in the chain. As used herein, aryl (ene) includes phenyl(ene) and alkaryl(ene). The alkyl (ene) or aryl(ene) may include heterocyclic groups. They may be such substituted such as hydroxyl, lower alkyl (i.e. up to $C_5$), amine, substituted amine (especially substituted with $C_{1-30}$, preferably $C_{1-5}$ alkyl groups), acyl, acyloxy, alkoxy, aryl, aroyl, aryloxy, aroyloxy, halogen, amido, imido groups, nitro, sulphonic acid and the like, they may be alkoxylated with ethers having $C_{1-30}$ alkyl groups, in particular, ethoxylated or propoxylated, or substituted as well as any other groups not adversely affecting the activity of the compound.

Where X is a leaving group, suitable leaving groups are for example any group which may be displaced from the bleach activator as a consequence of nucleophilic attack on the bleach activator. Generally leaving groups exert an electron attracting effect and are therefore weak bases. Where X is a leaving group it preferably forms a compound the conjugate acid of which has a pKa in the range 4-13, preferably 7-11, most preferably 8-11. Good leaving groups are also those which form a stable entity to minimise the rate of back reaction. Suitable leaving groups include

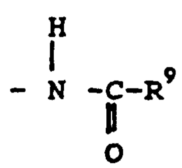

and

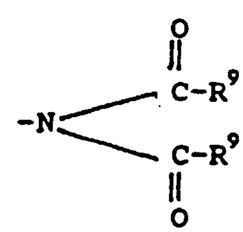

and preferred leaving groups include

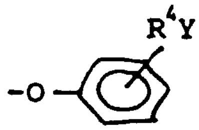

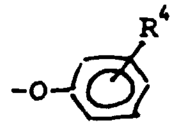

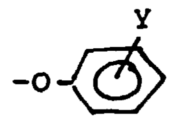

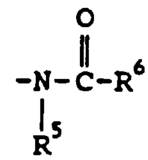

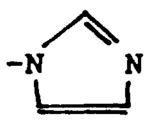

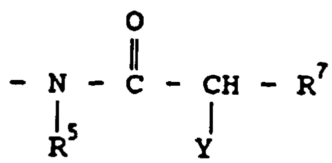

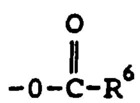

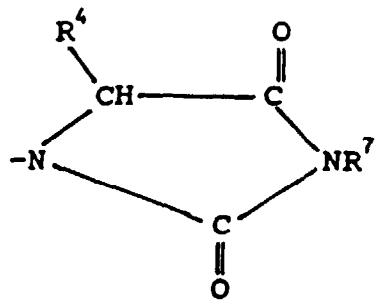

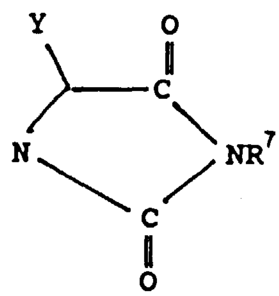

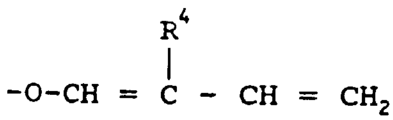

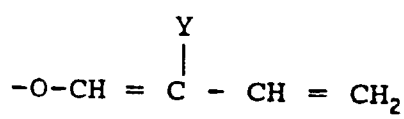

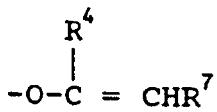 and 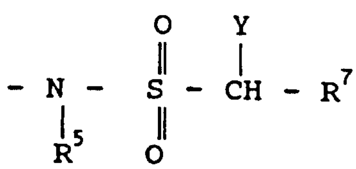

wherein $R^5$ and $R^6$ are hydrogen, or alkyl or aryl, preferably having from 1 to 14 carbon atoms;

$R^4$ is H or an alkyl chain containing from 1 to 8 carbon atoms;
$R^7$ is H, alkyl or aryl;
$R^9$ is alkyl or aryl, preferably having from 1-20, most preferably 1-14 carbon atoms; and
Y is H or a solubilising group.

The preferred solubilising groups in Y are $-SO_3^-M_1^+$, $-COO^-M_1^+$, $-SO_4^-M_1^+$,

$(-N^+R^8_4)X_2^-$ and 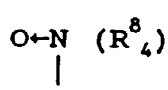

and most preferably $-SO_3^-M_1^+$ and $-COO^-M_1^+$ wherein $R^8$ is an alkyl chain containing from 1 to 4 carbon atoms, $M_1$ is a cation. Preferably, $M_1$ is a metal ion as described above (M), or ammonium or a substituted ammonium cation, with sodium and potassium being most preferred, and $X_2$ is a halide, hydroxide, methylsulfate or acetate anion.

On reaction of a bleach activator of the present invention with a peroxygen source, a more active bleaching species which may be a peracid is formed, in addition to unsubstituted heterocycle. Useful examples of bleach activators according to formula I include 5-membered ring heterocycles as defined in formula I where R is $\{CH_2\}_3$ and $R^1$ is a phenyl, 4-methylphenyl, 4-nitrophenyl, 4-tertiary butylphenyl, 1-ethylphenyl, 3-pyridyl, pentyl, 4-chlorophenyl, 4-methoxyphenyl, octyl, phenoxymethyl, 2-nitrophenyl, 3-nitrophenyl or tertiary butyl group;

7-membered ring heterocycles as defined in formula I where R is $\{CH_2\}_5$ and $R^1$ is 3,4 dicarboxyphenyl anhydride, tertiary butyl, 5-(N-phthalimidol)pentyl, phenoxymethyl,

8-membered ring heterocycles of formula I where R is $\{CH_2\}_6$ and $R^1$ is methyl or phenyl.

The present invention also includes a bleaching composition comprising one or mixtures of more than one of the bleach activators as defined above.

In the present invention the term "bleaching" is intended to include any process where the solution is used to remove or reduce unwanted colour in a substrate and/or reduce or remove non-coloured stains from fabric substrate and/or act as a disinfectant and the term "bleaching composition" is exemplified by but not limited to detergents, especially laundry detergents, dishwashing detergents, hard surface cleaners, including kitchen and bathroom cleaners, toilet cleaners, pipe cleaners, dry bleaches, compositions for bleaching wood, paper and pulp bleaching compositions, fabric and fibre bleaching compositions and oral hygiene compositions including mouthwashes, toothpastes and plaque-removing compositions and depilatory creams. In such bleaching compositions, there is included a precursor peroxygen source, that is a composition which releases perhydroxyl groups ($OOH^-$).

The precursor peroxygen source may be hydrogen peroxide itself, but is alternatively an inorganic salt for instance a percarbonate or a perborate. Sodium or potassium salts are preferred, such as sodium perborate, or an organic peroxide such as benzoyl peroxide or urea peroxide.

Alternatively, bleaching compositions according to the invention may be used in conjunction with separate compositions which comprise such a bleach precursor.

The invention also includes a process in which a peroxygen source is reacted with one of the above activator compounds in aqueous solution to form a product solution containing an oxidising product and the product solution is used as a bleach.

Where the peroxygen source is hydrogen peroxide itself, the concentration of hydrogen peroxide is preferably less than 70% (weight/volume) that is weight of hydrogen peroxide based on volume of water plus hydrogen peroxide plus other components in the mixture. Preferably the concentration is less than 60% weight by volume and more preferably less than 30% w/v. Where the product of the reaction is to be used in a domestic environment or other environment where it is difficult to take special precautions in handling the product, it is preferred for the concentration to be less than 15 or even less than 10% w/v. The concentration is usually at least 1% w/v more preferably at least 2% w/v.

Where the peroxygen source is other than hydrogen peroxide the concentration is preferably such as to give the equivalent available oxygen as the quoted concentrations of hydrogen peroxide. The concentration of peroxygen source in the aqueous liquid is for instance less than 10 M, preferably less than 5 M or sometimes even less than 3M down to 0.01 M. Preferably the concentration is at least 0.1M more preferably at least 0.2M. During the reaction, the temperature is preferably in the range 0 to 95°C, more preferably in the range 10 to 80°C, for instance in the range 20 to 60°C. The temperature in any subsequent oxidising step is preferably in the same ranges as the temperature during the reaction of the peroxygen source and the activator compound and is preferably substantially the same temperature specifically where the product solution is used for instance as a bleach or disinfectant. A particular advantage of using activators for the peroxygen source is that the oxidising product tends to be formed at a relatively low temperature, for instance 40°C or less, or even less than 30°C or 25°C which is advantageous for both safety and energy.

The compositions of the present invention are particularly useful in detergents, especially laundry detergents for both natural and synthetic fibres.

Generally, the reaction between the peroxygen source and the activator will take place under alkaline conditions, above pH 7 or 7.5. However, the reaction may also take place under acidic conditions. Generally the pH for the reaction will be from 6 to 14. Preferably it will be above 7.5 or even 8 and most preferably the pH will be from 9.5 to 12.5. Generally the bleaching compositions will be alkaline at a pH above 7.5. However, the composition may also contain a pH-modifier which may comprise an acid, a base and/or buffering material so that the desired pH can be obtained for the reaction between the precursor and the activator. For example, when the pH-modifier is an acidifying pH-modifier, it may comprise a polybasic organic acid such as a polybasic carboxylic acid, for example citric, succinic, adipic or sulphonic acid. Alternatively the pH-modifier may react with a by-product of the perhydrolysis reaction of the peroxygen source with the activator compound to produce an acid or base. Where perborate is used, borate is a by-product and so any component known to react with perborate to produce acidity for example a polyol, boric acid or sodium di-hydrogen phosphate can be used to provide the pH-modifier.

In the composition of the present invention, the peroxygen source and the activator compound may be provided in separate compositions. For instance, the peroxygen source may be in the form of an aqueous solution of

peroxygen whilst the activator may be provided in the solid form. However, preferably the components are provided in a stable composition, which is preferably therefore substantially water free.

The composition may be in liquid form, for instance in a non-aqueous liquid medium, in which the components may be dissolved or dispersed. For instance particles of activator with protective coatings, for instance produced by micro-encapsulation techniques or spray coating of solid activator, may be suspended in an aqueous, or non aqueous, solution of peroxygen source. As an alternative to a solution of peroxygen source that component may also be suspended in the liquid medium, either in a separate liquid phase or in particulate dispersed phase, particles of solid peroxygen source optionally being coated with a protective coating. Coated particles of either peroxygen source or activator may be disrupted or diluted into water or with abrasion.

Preferably the composition (or compositions) is in solid form, for instance as a mixture of particles of the individual components or, more preferably, comprising particles each of which comprise all of the components. Such particles may be provided by techniques the same or similar to those conventionally used in the laundry detergent industry, for instance including particles produced by spray drying liquid slurries, by granulation techniques using binders (for instance synthetic or natural polymers or derivatives) or by melt blending followed by extrusion or other techniques.

Preferably the composition contains the active ingredients in appropriate relative quantities so that when the composition is diluted (or the compositions are mixed) with water the first step of the reaction proceeds at the optimal rate and at the desired pH. The activator and peroxygen source are for instance present in relative amounts such that 5% to 150% of the stoichiometric amount of activator (for complete reaction with the peroxygen source) is provided. Preferably the amount of activator is 10 to 100%, more preferably 20 to 80% of the stoichiometric amount.

The new composition may include other additives, for instance stabilisers which stabilise the product before use, as well as stabilisers for the peroxy acid oxidising species formed in the reaction, especially heavy metal sequestrants. The new product may also include surfactants to act as wetting agents and inorganic salts, for instance which affect the physical properties of the solid form or act as diluent. Other ingredients may be included depending on the final application of the reaction product, for instance perfumes, or agents to assist dissolution or dispersion of the product into water.

The N-substituted heterocyclic bleach activator of formula I may be simply admixed directly in with the other detergent composition compounds. Alternatively it could be coformulated with builders (for example phosphates, polyphosphates, sodium carbonate, silicates, zeolites clays, borax) complexing or sequestering agents (ethylenediamine tetraacetic acid, diethylenepentamine pentaacetic acid, nitrilotriacetic acid, citrates, phosphonates), enzymes, surfactants (linear alkylbenzene sulphonates, phenol sulphonates, alcohol ether sulphates, alcohol ethoxylates, amphoteric and cationic surfactants), soap, polyacrylates, hydrotropes, mineral oil and foam regulators.

The reaction product of the peroxygen source and the activator is preferably used immediately, without removal of any by-products or addition of other materials. Sometimes it may be desirable to add additional ingredients to the composition after the reaction between the peroxygen source and bleach activator such as pH-adjusters, wetting agents, additives to improve the action of the bleaching agent produced in the reaction, for instance co-disinfectants, biocides, abrasives etc.

The invention has been found to be surprisingly advantageous when the composition of the present invention comprises a peroxygen source and an N-substituted heterocycle of formula I as defined above, in combination with a conventional activator. The compositions have been found to exhibit enhanced bleaching effects. The compositions are especially effective as laundry detergents for both natural and synthetic fibres. Suitable conventional activators include any compound or mixture of compounds already known as useful bleach activators. Particularly preferred conventional activators are tetra-acylated alkylene diamines such as TAMD or TAED, tetraacylated glycol urils especially TAGU (tetraacetyl glycol uril), pentaacylated glucose, especially PAG (pentaacetyl glucose) and acylated (preferably $C_{6-14}$) oxybenzene sulphonates, especially SNOBS (sodium nonanoyl oxybenzene sulphonate).

It has been found that compositions incorporating both one or more of the N-acyl caprolactam activators of the present invention and a tetraacylated alkylene diamine, preferably TAED have particularly improved detergency properties.

In accordance with a further aspect of the present invention there is provided a bleaching composition comprising a peroxygen source, a conventional bleach activator, preferably a tetraacylated alkylene diamine and a bleach activator of the formula I as defined in claim 1.

Preferably $R^1$ is a $C_{7-30}$ most preferably $C_{7-15}$ group, or has the formula $-R^2-COX$ where X is as defined above and $R^2$ is a $C_{7-30}$ group. Preferably $R^1$ or $R^2$ is straight chain alkyl(ene) or aryl(ene) group. Preferably R is a $C_5$ group or a $C_3$ group. Preferably R is an unsubstituted alkylene group, eg. $-(CH_2)_5$ or $(CH_2)_3$.

A particularly preferred $R^1$ or $R^2$ group is an optionally substituted aryl(ene) group especially substituted phenyl(ene) group. A further preferred group includes bleach activators having the formula I above and in which $R^1$ represents a $C_{10-30}$ or has the formula $-R^2-COX$ where X is as defined above, $R^2$ is a $C_{11-30}$ alkyl group and R comprises from 2 to 7-$CH_2$- groups. Without wishing to be bound by theory, it is postulated that the particular advantages which

have been found for a combination of the N-acyl bleach activators of formula I with acylated alkylene diamines, and in particular TAED, are due to the short chain nature of the peroxygen bleach compound produced using the alkylene diamine resulting in good hydrophilicity and the relatively long chain peroxygen bleach compounds formed from the activator of the present invention producing good hydrophobicity. Thus, the combination of the two activators used in combination gives good cleaning properties.

In a further embodiment of the invention, the conventional activator to be used in combination with the activator as defined in formula I above comprises SNOBS (sodium nonanoyloxy benzene sulphate). In this embodiment of the invention, it is particularly preferred that the SNOBS is in combination with a bleach activator having the formula I above, in which $R^1$ represents a $C_{1-9}$, more preferably $C_{2-6}$ alkyl group or has the formula $-R^2-COX$ where X is as defined above and $R^2$ is a $C_{1-10}$ alkyl(ene) group, preferably a $C_{2-6}$ alkyl group and R is a $C_{2-7}$ group, preferably alkylene in which the $CH_2$ groups of the lactam are unsubstituted.

Where the composition comprises at least two bleach activators, the first being a bleach activator of formula I, preferably the activator of formula I is present in an amount of at least 50% by weight total bleach activator, more preferably at least 70%, or even at least 80 or 90% by weight total bleach activator.

The invention is particularly directed to the manufacture of detergents for example used in washing laundry and the compositions therefore preferably include conventional surfactants.

The novel bleach activators of the present invention can be prepared by any conventional organic synthesis route. They may be prepared by reacting the unsubstituted heterocycle with an acid chloride in which the acyl group is the desired acyl group for N-substitution on the unsubstituted heterocycle. The reaction is preferably carried out under alkaline conditions in a suitable organic solvent such as toluene. The reaction product can be crystallised or precipitated out of the solvent medium.

The invention is illustrated by way of the following examples:

**Example 1**

**Preparation of 4-nitrobenzoyl 2-pyrrolidone.**

A two litre flat-flanged flask equipped with thermometer, stirrer, dropping funnel and reflux condenser was charged with 292.0g of toluene, 2.78 mol of 2-pyrrolidone and 3.00 mol triethylamine. Separately, 2.78 mol 4-nitrobenzoyl chloride was dissolved in 400g of toluene and this mixture was added drop-wise to the contents of the reaction flask at room temperature. Once the addition was completed, the contents of the flask were refluxed for 4 hours. The reaction mixture was then cooled to room temperature and washed with 2 x 100cm$^3$ water to remove the amine hydrochloride. The mixture was then filtered to give the first crop of product. A second crop of product may be obtained from the toluene layer if required by recrystallisation. The yield of 4-benzoyl 2-pyrrolidone was 474.2g (72.9%). The melting point of the product was 127-129°C and the purity as measured using HPLC was 98.7%. An NMR spectrum of the product was obtained and the results were as follows:

(1) doublet of doublets at around 8ppm (8.25 ppm and 7.70 ppm) indicative of 4-di-substituted aromatic hydrogen atoms ($H_7$-$H_{10}$).
(2) quintuplet centred at around 2.17ppm ($H_3$ and $H_4$).
(3) triplet centred at 3.98 ppm ($H_1$ and $H_2$).
(4) triplet centred at 2.62 ppm ($H_5$ and $H_6$).

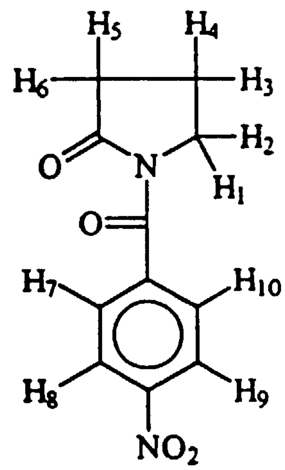

An IR spectrum of the product was obtained giving results: 2 bands at 1746$cm^{-1}$ and 1666$cm^{-1}$, respectively, which are indicative of the two carbonyl bonds associated with cyclic imides.

**Performance Testing**

The bleach activator prepared in example 1 and several other bleach activators of the present invention as listed in Table 1, were tested for washing performance, comparisons being made using: (i) a blank incorporating no bleach activator and (ii) a comparative known bleach activator.

Swatches of 100% cotton 12cm x 12cm were stained using the stains listed below:

| WIL Tea | Tea prepared by Warwick International Group Limited |
|---|---|
| BC1 | Tea stains with clay |
| BC2 | Coffee stains |
| BC3 | Tea stains |
| BC4 | Curry stains |
| AS4 | Chlorophyll stains |

These stains are useful to give an indication of the bleaching power of formulations incorporating bleach activator. AS4 is particularly good at indicating bleaching performance on oily stains.

Wash tests were carried out under conditions typical of both European and U.S. laundry washing conditions. European wash tests were carried out at each of 40°C and 60°C, using cold fill Wascator FOM 71 MP machines, programmed to BS 4923 (HLCC) wash programmes. U.S. wash tests were carried out at 40°C only, using Maytag (A7500 Model) or Whirlpool machines (Super Capacity Model 3LA5580XS).

For the European wash tests, formulations incorporating the activator for testing were prepared containing 22.5g PBS4 (sodium perborate tetrahydrate), 124.5g IEC standard base detergent and a weight of activator calculated to give the same moles peracid release as 2% by weight TAED. Formulations were dosed at 7.5g/l wash water via the dispensing drawer of the machines.

For the U.S. wash tests a wash cycle of 12 minutes duration was used, the temperature of the incoming water being 40°C. Formulations contained 5% by weight activator for testing (100% active) 10% PBS1 (sodium perborate monohydrate) and IEC to 100%. The formulations were dosed at 1.5g/l.

During the wash tests the swatches were tagged to 2.5kg polyester backing cloth, using plastic tags. After washing the swatches were removed and ironed dry prior to reading reflectance measurements to assess stain removal. Stain removal was assessed as a percentage brightness and calculated using the equation.

$$\text{Stain removal} = \frac{(RA - RB)}{(RS - RB)} \times 100$$

where RA is the reflectance of the washed swatch, RB is the reflectance of the unwashed swatch and RS is the

reflectance of an unstained swatch. Reflectance was measured using a Spectroflash 500 Spectrophotometer machine.

Due to variations in swatch make up all evaluations were run compared to a TAED or NBC (N-benzoyl caprolactam) or other conventional activator standard, as well as a blank incorporating no activator.

Table 1

| Compound | R Group | $R^1$ Group | M.Pt(°C) | Purity (HPLC) |
|---|---|---|---|---|
| A | $(CH_2)_3$ | phenyl | 80-91 | 99.7 |
| B | $(CH_2)_3$ | 4-methylphenyl | 130-131 | 99.8 |
| C | $(CH_2)_3$ | 4-nitrophenyl | 130-131 | 97.3 |
| D | $(CH_2)_3$ | 4-tert-butylphenyl | 104.8-105.9 | 99.4 |
| E | $(CH_2)_3$ | pentyl | liquid | 88.1 |
| F | $(CH_2)_3$ | 4-chlorophenyl | 112.5-114.5 | 99.3 |
| G | $(CH_2)_3$ | 4-methoxyphenyl | 119.7-120.1 | 98.8 |
| H | $(CH_2)_3$ | octyl | liquid | 97.3 |
| I | $(CH_2)_3$ | phenoxymethyl | - | 99.6 |
| J | $(CH_2)_5$ | 3,4-dicarboxyphenyl anhydride | - | - |
| L | $(CH_2)_5$ | tert-butyl | liquid | 95 |
| M | $(CH_2)_5$ | 5-(N-phthalimidol) pentyl | gel | 82.9 |
| N | $(CH_2)_5$ | phenoxymethyl | 75 | 99.6 |
| O | $(CH_2)_6$ | phenyl | - | 97.4 |

The results for the wash tests carried out on each of the bleach activators listed in table 1 above, are given in the tables below. In order to ensure that any effect on the results obtained due to variations in water quality could be minimised, for each set of tests run at the same time, a standard and a blank test were also run. The results in the tables below are given in blocks of tests which were carried out at the same time.

The results in tables 2 to 4 below illustrate the effective bleaching properties obtained using the bleach activators of the present invention under the washing conditions tested.

TABLE 2.

| **Compound** | **Wash Test Results (European Washes @ 40°C)** | | | | | |
|---|---|---|---|---|---|---|
| | **WIL TEA** | **BC1** | **BC2** | **BC3** | **BC4** | **AS4** |
| Blank | 27.9 | 4.0 | 11.9 | 3.7 | 12.4 | 31.3 |
| TAED | 32.8 | 9.0 | 17.4 | 13.8 | 12.9 | 32.0 |
| NBC | 37.8 | 13.4 | 24.0 | 18.6 | 14.5 | 36.2 |
| A | 32.5 | 10.5 | 21.7 | 16.4 | 16.3 | 34.6 |
| B | 31.7 | 11.2 | 22.6 | 20.2 | 13.8 | 39.9 |
| C | 38.6 | 14.6 | 24.9 | 24.0 | 16.3 | 36.9 |
| | | | | | | |
| Blank | 32.0 | 8.0 | 14.0 | 12.9 | 22.5 | 22.6 |
| TAED | 33.6 | 10.0 | 17.8 | 20.8 | 21.2 | 22.3 |
| NBC | 41.4 | 13.1 | 23.0 | 28.0 | 25.3 | 25.7 |
| D | 31.3 | 10.0 | 20.2 | 21.3 | 22.1 | 30.7 |
| | | | | | | |
| Blank | 29.5 | 4.9 | 5.4 | 8.8 | 11.6 | 16.5 |

TABLE 2. (continued)

| Compound | Wash Test Results (European Washes @ 40°C) | | | | | |
|---|---|---|---|---|---|---|
| | WIL TEA | BC1 | BC2 | BC3 | BC4 | AS4 |
| TAED | 32.6 | 5.7 | 7.1 | 13.9 | 12.0 | 17.1 |
| NBC | 32.2 | 9.6 | 8.7 | 16.7 | 14.0 | 19.1 |
| E | 33.6 | 6.1 | 7.0 | 13.0 | 10.1 | 19.0 |
| F | 36.0 | 10.7 | 10.7 | 20.4 | 13.6 | 23.5 |
| | | | | | | |
| Blank | 25.7 | 12.7 | 12.2 | 23.2 | 13.3 | 19.2 |
| TAED | 29.4 | 12.0 | 12.9 | 22.6 | 13.4 | 15.5 |
| NBC | 31.9 | 15.2 | 14.9 | 24.9 | 12.6 | 15.9 |
| G | 26.8 | 15.4 | 13.8 | 26.9 | 14.9 | 19.5 |
| H | 24.1 | 8.5 | 10.2 | 19.8 | 7.1 | 17.0 |
| | | | | | | |
| Blank | 35.2 | 4.1 | 5.1 | 13.6 | 10.7 | 25.1 |
| TAED | 40.9 | 8.4 | 8.6 | 21.6 | 10.3 | 24.9 |
| NBC | 39.6 | 8.7 | 8.6 | 22.6 | 13.5 | 27.6 |
| I | 32.1 | 7.4 | 6.4 | 20.6 | 12.2 | 22.2 |
| | | | | | | |
| Blank | 30.1 | 3.6 | 11.8 | 5.0 | 13.6 | 33.3 |
| TAED | 33.6 | 8.4 | 17.6 | 13.8 | 13.6 | 32.5 |
| J | 38.4 | 8.0 | 16.7 | 9.9 | 8.4 | 33.4 |
| | | | | | | |
| Blank | 32.0 | 8.0 | 14.0 | 12.9 | 22.5 | 22.6 |
| TAED | 33.6 | 10.0 | 17.8 | 20.8 | 21.2 | 22.3 |
| NBC | 41.4 | 13.1 | 23.0 | 28.0 | 25.3 | 25.7 |
| L | 32.0 | 10.7 | 19.4 | 20.3 | 22.5 | 24.5 |
| | | | | | | |
| Blank | 25.7 | 12.7 | 12.2 | 23.2 | 13.3 | 35.1 |
| TAED | 29.4 | 12.0 | 12.9 | 22.6 | 13.4 | 24.8 |
| NBC | 31.9 | 15.2 | 14.9 | 24.9 | 12.6 | 15.9 |
| M | 22.7 | 10.7 | 10.6 | 18.3 | 12.2 | 27.3 |
| | | | | | | |
| Blank | 35.2 | 4.1 | 5.1 | 13.6 | 10.7 | 25.1 |
| TAED | 40.9 | 8.4 | 8.6 | 21.6 | 10.3 | 24.9 |
| SNOBS | 39.6 | 8.7 | 8.6 | 22.6 | 13.5 | 27.6 |
| N | 38.6 | 12.6 | 11.3 | 29.9 | 12.6 | 26.3 |
| | | | | | | |
| Blank | 35.2 | 4.1 | 5.1 | 13.6 | 10.7 | 25.1 |
| TAED | 40.9 | 8.4 | 8.6 | 21.6 | 10.3 | 24.9 |

TABLE 2. (continued)

| Compound | Wash Test Results (European Washes @ 40°C) | | | | | |
|---|---|---|---|---|---|---|
| | WIL TEA | BC1 | BC2 | BC3 | BC4 | AS4 |
| NBC | 39.6 | 8.7 | 8.6 | 22.6 | 13.5 | 27.6 |
| O | 38.0 | 10.2 | 8.2 | 23.9 | 10.3 | 26.1 |
| | | | | | | |

TABLE 3

| Compound | Wash Test Results (European Washes @ 60°C) | | | | | |
|---|---|---|---|---|---|---|
| | WIL TEA | BC1 | BC2 | BC3 | BC4 | AS4 |
| Blank | 38.6 | 10.5 | 18.6 | 16.1 | 26.3 | 44.6 |
| TAED | 48.7 | 18.2 | 29.4 | 27.8 | 25.4 | 46.4 |
| NBC | 44.5 | 21.2 | 31.9 | 31.1 | 33.2 | 49.0 |
| A | 43.1 | 17.6 | 31.7 | 28.5 | 31.7 | 49.7 |
| B | 47.3 | 21.2 | 36.7 | 32.2 | 30.1 | 52.4 |
| C | 53.3 | 24.5 | 40.0 | 37.4 | 33.1 | 53.9 |
| | | | | | | |
| Blank | 43.0 | 12.2 | 23.1 | 29.5 | 31.8 | 32.3 |
| TAED | 54.8 | 18.9 | 30.8 | 37.5 | 34.9 | 35.0 |
| NBC | 54.7 | 20.3 | 35.8 | 41.9 | 39.6 | 41.8 |
| D | 46.6 | 18.9 | 35.2 | 38.3 | 42.5 | 49.6 |
| | | | | | | |
| Blank | 40.1 | 10.7 | 11.3 | 23.1 | 21.3 | 22.5 |
| TAED | 45.4 | 14.1 | 14.4 | 30.4 | 23.7 | 23.9 |
| NBC | 46.7 | 15.3 | 16.7 | 35.5 | 24.4 | 27.1 |
| E | 48.2 | 13.3 | 14.7 | 33.2 | 23.3 | 25.9 |
| F | 46.0 | 17.2 | 18.3 | 38.9 | 26.2 | 30.8 |
| | | | | | | |
| Blank | 31.9 | 19.3 | 15.0 | 31.7 | 19.7 | 29.7 |
| TAED | 40.1 | 21.8 | 20.2 | 37.2 | 20.2 | 29.4 |
| NBC | 41.3 | 21.4 | 22.2 | 38.1 | 24.8 | 33.9 |
| G | 40.3 | 22.8 | 23.7 | 37.7 | 26.4 | 31.7 |
| H | 31.9 | 18.7 | 17.2 | 30.0 | 22.3 | 30.4 |
| | | | | | | |
| Blank | 36.9 | 11.1 | 9.8 | 28.1 | 22.2 | 29.9 |
| TAED | 43.3 | 12.4 | 12.5 | 34.2 | 22.0 | 31.5 |
| NBC | 47.7 | 16.2 | 16.3 | 40.1 | 26.8 | 34.8 |
| I | 40.3 | 11.8 | 12.5 | 32.6 | 20.6 | 32.6 |
| | | | | | | |
| Blank | 38.5 | 10.6 | 21.9 | 19.2 | 25.1 | 46.3 |

TABLE 3 (continued)

| Compound | Wash Test Results (European Washes @ 60°C) | | | | | |
|---|---|---|---|---|---|---|
| | WIL TEA | BC1 | BC2 | BC3 | BC4 | AS4 |
| TAED | 50.3 | 17.6 | 31.1 | 28.8 | 27.6 | 44.3 |
| J | 49.1 | 11.4 | 23.0 | 19.5 | 24.1 | 43.2 |
| | | | | | | |
| Blank | 43.0 | 12.2 | 23.1 | 29.5 | 31.8 | 32.3 |
| TAED | 54.8 | 18.9 | 30.8 | 37.5 | 34.9 | 35.0 |
| NBC | 54.7 | 20.3 | 35.8 | 41.9 | 39.6 | 41.8 |
| L | 51.5 | 18.4 | 32.9 | 38.5 | 37.2 | 38.7 |
| | | | | | | |
| Blank | 31.9 | 19.3 | 15.0 | 31.7 | 19.7 | 47.4 |
| TAED | 40.1 | 21.8 | 20.2 | 37.2 | 20.2 | 42.8 |
| SNOBS | 41.3 | 21.4 | 22.2 | 38.1 | 24.8 | 33.9 |
| M | 34.3 | 17.0 | 16.2 | 29.0 | 19.6 | 46.4 |
| | | | | | | |
| Blank | 36.9 | 11.1 | 9.8 | 28.1 | 22.2 | 29.9 |
| TAED | 43.3 | 12.4 | 12.5 | 34.2 | 22.0 | 31.5 |
| SNOBS | 47.7 | 16.2 | 16.3 | 40.1 | 26.8 | 34.8 |
| N | n/t | n/t | n/t | n/t | n/t | n/t |
| | | | | | | |
| Blank | 36.9 | 11.1 | 9.8 | 28.1 | 22.2 | 29.9 |
| TAED | 43.3 | 12.4 | 12.5 | 34.2 | 22.0 | 31.5 |
| NBC | 47.7 | 16.2 | 16.3 | 40.1 | 26.8 | 34.8 |
| O | 45.5 | 15.3 | 15.3 | 36.0 | 25.7 | 35.0 |
| | | | | | | |

TABLE 4

| Compound | Wash Test Results (US Washes @ 40°C) | | | | | |
|---|---|---|---|---|---|---|
| | WIL TEA | BC1 | BC2 | BC3 | BC4 | AS4 |
| Blank | 10.1 | 0.2 | 5.0 | 0.7 | 7.7 | 7.3 |
| TAED | 15.5 | 3.7 | 6.3 | 2.4 | 8.9 | 8.4 |
| NBC | 13.0 | 4.3 | 8.8 | 2.2 | 8.4 | 15.3 |
| A | 15.0 | 4.4 | 8.2 | 2.2 | 10.9 | 13.6 |
| B | 9.8 | 4.3 | 8.5 | 3.2 | 10.2 | 15.1 |
| C | 15.1 | 4.9 | 8.9 | 4.1 | 11.1 | 11.5 |
| | | | | | | |
| Blank | 10.9 | 2.3 | 2.9 | -2.3 | 18.6 | 5.3 |
| TAED | 17.0 | 2.9 | 6.3 | 0.3 | 15.3 | 5.9 |
| NBC | 11.8 | 4.6 | 7.9 | -0.5 | 17.8 | 5.6 |

TABLE 4 (continued)

| Compound | Wash Test Results (US Washes @ 40°C) | | | | | |
|---|---|---|---|---|---|---|
| | WIL TEA | BC1 | BC2 | BC3 | BC4 | AS4 |
| D | 7.3 | 3.9 | 5.6 | -1.8 | 19.1 | 8.8 |
| | | | | | | |
| Blank | 21.0 | 2.3 | 3.1 | 6.2 | 8.3 | 11.0 |
| TAED | 28.3 | 3.8 | 3.9 | 9.4 | 7.6 | 11.3 |
| SNOBS | 23.4 | 4.3 | 5.7 | 11.9 | 11.1 | 14.6 |
| E | 25.7 | 4.4 | 3.8 | 4.6 | 7.7 | 12.2 |
| F | 24.0 | 6.1 | 4.5 | 10.3 | 8.2 | 13.0 |
| | | | | | | |
| Blank | 17.6 | 5.9 | 5.9 | 8.6 | 6.6 | 9.1 |
| TAED | 23.5 | 11.3 | 7.9 | 9.2 | 7.8 | 12.5 |
| SNOBS | 18.5 | 5.5 | 6.9 | 9.0 | 13.4 | 12.0 |
| G | 19.4 | 10.0 | 8.8 | 9.2 | 6.3 | 5.0 |
| H | 19.0 | 8.8 | 5.9 | 5.8 | 9.7 | 9.3 |
| | | | | | | |
| Blank | 25.8 | 1.6 | 2.4 | 6.4 | 7.2 | 19.4 |
| TAED | 32.0 | 4.5 | 2.9 | 7.8 | 10.2 | 19.1 |
| SNOBS | 29.3 | 6.1 | 5.8 | 10.0 | 9.2 | 25.7 |
| I | 29.3 | 3.5 | 3.3 | 6.3 | 11.9 | 20.0 |
| | | | | | | |
| Blank | 8.6 | 0.3 | 6.9 | 1.7 | 11.2 | 9.9 |
| TAED | 17.6 | 3.4 | 4.7 | 2.2 | 11.4 | 10.9 |
| SNOBS | 17.3 | 5.0 | 11.5 | 1.5 | 18.8 | 21.9 |
| J | 17.3 | 2.3 | 5.2 | 1.9 | 9.7 | 8.4 |
| | | | | | | |
| Blank | 10.9 | 2.3 | 2.9 | -2.3 | 18.6 | 5.3 |
| TAED | 17.0 | 2.9 | 6.3 | 0.3 | 15.3 | 5.9 |
| NBC | 11.8 | 4.6 | 7.9 | -0.5 | 17.8 | 5.6 |
| L | 5.5 | 3.2 | 4.5 | -0.7 | 14.1 | 6.4 |
| | | | | | | |
| Blank | 17.6 | 5.9 | 5.9 | 8.6 | 6.6 | 9.1 |
| TAED | 23.5 | 11.3 | 7.9 | 9.2 | 7.8 | 12.5 |
| SNOBS | 18.5 | 5.5 | 6.9 | 9.0 | 13.4 | 12.0 |
| M | 11.2 | 6.8 | 6.9 | 7.2 | 3.9 | 2.7 |
| | | | | | | |
| Blank | 25.8 | 1.6 | 2.4 | 6.4 | 7.2 | 19.4 |
| TAED | 32.0 | 4.5 | 2.9 | 7.8 | 10.2 | 19.1 |
| SNOBS | 29.3 | 6.1 | 5.8 | 10.0 | 9.2 | 25.7 |

TABLE 4 (continued)

| Compound | Wash Test Results (US Washes @ 40°C) | | | | | |
|---|---|---|---|---|---|---|
| | WIL TEA | BC1 | BC2 | BC3 | BC4 | AS4 |
| N | 26.5 | 4.5 | 2.5 | 6.0 | 9.1 | 18.6 |
| | | | | | | |
| Blank | 25.8 | 1.6 | 2.4 | 6.4 | 7.2 | 19.4 |
| TAED | 32.0 | 4.5 | 2.9 | 7.8 | 10.2 | 19.1 |
| SNOBS | 29.3 | 6.1 | 5.8 | 10.0 | 9.2 | 25.7 |
| O | 23.8 | 1.9 | -1.1 | 4.1 | 7.9 | 19.4 |

## Claims

1. A composition containing a peroxygen source and a bleach activator comprising an optionally substituted N-acyl heterocycle having the formula I:

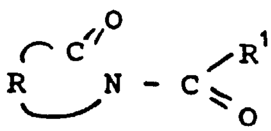

(I)

in which R represents an optionally substituted $C_3$, $C_5$ or $C_6$-alkylene function which forms a cyclic structure with - N- and -C(O)- and,

where R represents a $C_3$ or $C_6$ alkylene, $R^1$ represents a $C_{1-30}$ alkyl or aryl group; or has the formula $-R^2-COX$ where X may be OH, OM (where M is a metal ion), $-ON^+R^3_3$ (where $R^3$ are each independently selected from H and $C_{1-4}$ alkyl), a halogen atom, amine, $C_{1-30}$ alkyl or alkoxy or a leaving group and $R^2$ is a $C_{1-30}$ alkylene or arylene group disclaiming compounds in which R is $C_3$-alkylene which has a $COOR^x$ substituent at the carbon atom joined to the ring nitrogen atom, where $R^x$ is hydrogen, $C_{1-4}$-alkyl, alkali metal or ammonium;

where R represents $C_5$ alkylene, it is $(CH_2)_5$ and $R^1$ is selected from 3,4-dicarboxyphenylanhydride, 5-(N-phthalimidoyl) pentyl, phenoxymethyl and tertiary butyl.

2. A composition according to claim 1 in which R is $(CH_2)_3$.

3. A composition according to claim 2 in which $R^1$ is an optionally substituted phenyl group.

4. A composition according to claim 3 in which $R^1$ is selected from phenyl, 4-methylphenyl, 4-nitrophenyl, 4-tertiarybutylphenyl, 4-chlorophenyl, 4-methoxyphenyl.

5. A composition according to claim 2 in which $R^1$ is pentyl, octyl or phenoxymethyl.

6. A composition according to claim 1 in which R is $(CH_2)_5$.

7. A composition according to claim 1 in which R is $C_6$-alkylene.

8. A composition according to claim 7 in which $R^1$ is an optionally substituted phenyl group.

9. A composition according to any preceding claim which is a laundry detergent.

10. A bleaching composition according to any preceding claim which comprises at least one further bleach activator in admixture with the said N-acyl heterocyclic compound.

11. A composition according to claim 10 in which the further bleach activator comprises a tetra-acylated ethylene

diamine, preferably tetraacetylethylene diamine.

**12.** A bleaching composition according to claim 10 in which the further bleach activator comprises sodium nonanoyloxybenzene sulphonate.

**13.** A process in which a peroxygen source is reacted with an activator compound as defined in any of claims 1 to 8 in a perhydrolysis step in an aqueous solution to form a product solution containing an oxidised product and the product solution is used as a bleach.

**Patentansprüche**

**1.** Zusammensetzung, enthaltend eine Persauerstoff-Quelle und einen Bleichmittel-Aktivator, welcher einen gegebenenfalls substituierten N-Acyl-Heterocyclus mit der Formel I umfaßt:

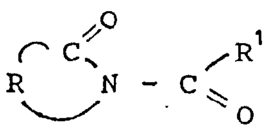

(I)

worin R für eine gegebenenfalls substituierte $C_3$-, $C_5$- oder $C_6$-Alkylenfunktion steht, die mit -N- und -C(O)- eine cyclische Struktur bildet, und
wenn R ein $C_3$- oder $C_6$-Alkylen bedeutet, $R^1$ für eine $C_{1-30}$-Alkyl- oder -Arylgruppe steht; oder die Formel -$R^2$-COX hat, worin X OH, OM (worin M ein Metallion ist), $-ON^+R^3_3$ (worin $R^3$ jeweils unabhängig ausgewählt ist unter H und $C_{1-4}$-Alkyl), ein Halogenatom, Amin, $C_{1-30}$-Alkyl oder -Alkoxy oder eine Abgangsgruppe sein kann und $R^2$ eine $C_{1-30}$-Alkylen- oder -Arylengruppe ist, wobei Verbindungen ausgenommen sind, in denen R ein $C_3$-Alkylen ist, das einen $COOR^x$-Substituenten an dem Kohlenstoffatom aufweist, welches an das Stickstoffatom des Rings gebunden ist, wobei $R^x$ für Wasserstoff, $C_{1-4}$-Alkyl, Alkalimetall oder Ammonium steht;
wenn R $C_5$-Alkylen bedeutet, es sich um $(CH_2)_5$ handelt und $R^1$ ausgewählt ist unter 3,4-Dicarboxyphenylanhydrid, 5-(N-Phthalimidoyl)pentyl, Phenoxymethyl und tert.-Butyl.

**2.** Zusammensetzung nach Anspruch 1, worin R $(CH_2)_3$ ist.

**3.** Zusammensetzung nach Anspruch 2, worin $R^1$ eine gegebenenfalls substituierte Phenylgruppe ist.

**4.** Zusammensetzung nach Anspruch 3, worin $R^1$ ausgewählt ist unter Phenyl, 4-Methylphenyl, 4-Nitrophenyl, 4-tert.-Butylphenyl, 4-Chlorphenyl, 4-Methoxyphenyl.

**5.** Zusammensetzung nach Anspruch 2, worin $R^1$ Pentyl, Octyl oder Phenoxymethyl ist.

**6.** Zusammensetzung nach Anspruch 1, worin R $(CH_2)_5$ ist.

**7.** Zusammensetzung nach Anspruch 1, worin R $C_6$-Alkylen ist.

**8.** Zusammensetzung nach Anspruch 7, worin $R^1$ eine gegebenenfalls substituierte Phenylgruppe ist.

**9.** Zusammensetzung nach irgendeinem vorhergehenden Anspruch, bei welcher es sich um ein Waschmittel handelt.

**10.** Bleichzusammensetzung nach irgendeinem vorhergehenden Anspruch, welche mindestens einen weiteren Bleichmittel-Aktivator in Mischung mit der heterocyclischen N-Acyl-Verbindung umfaßt.

**11.** Zusammensetzung nach Anspruch 10, worin der weitere Bleichmittel-Aktivator ein tetra-acyliertes Ethylendiamin, vorzugsweise Tetraacetylethylendiamin, umfaßt.

**12.** Bleichzusammensetzung nach Anspruch 10, worin der weitere Bleichmittel-Aktivator Natriumnonanoyloxybenzolsulfonat umfaßt.

**13.** Verfahren, in welchem eine Persauerstoff-Quelle mit einer wie in irgendeinem der Ansprüche 1 bis 8 definierten Aktivator-Verbindung in einem Perhydrolyse-Schritt in einer wäßrigen Lösung umgesetzt wird, um eine Produktlösung zu bilden, die ein oxidiertes Produkt enthält, und die Produktlösung als Bleichmittel verwendet wird.

## Revendications

**1.** Composition contenant une source de peroxygène et un activateur de blanchiment comprenant un hétérocycle N-acylé éventuellement substitué ayant la formule I:

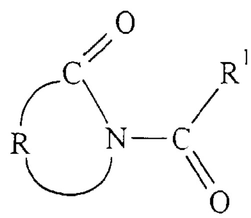

(I)

dans laquelle R représente une fonction alkylène en $C_3$, $C_5$ ou $C_6$ éventuellement substituée, qui forme une structure cyclique avec -N- et -C(O)- et,

lorsque R représente alkylène en $C_3$ ou $C_6$, $R^1$ représente un groupe alkyle en $C_{1-30}$ ou aryle ; ou est un groupe de formule $-R^2$-COX, dans laquelle X peut être OH, OM (où M est un ion métallique), $-ON^+R^3_3$ (où chaque $R^3$ est choisi indépendamment parmi H et alkyle en $C_{1-4}$), un atome d'halogène, un groupe amine, alkyle en $C_{1-30}$ ou alcoxy en $C_{1-30}$ ou un groupe partant et $R^2$ est un groupe alkylène en $C_{1-30}$ ou arylène, à l'exception des composés dans lesquels R est alkylène en $C_3$ qui comporte un substituant $COOR^x$ sur l'atome de carbone lié à l'atome d'azote cyclique, où $R^x$ est hydrogène, alkyle en $C_{1-4}$, un métal alcalin ou un ammonium;

lorsque R représente alkylène en $C_5$, il s'agit de $-(CH_2)_5-$ et $R^1$ est choisi parmi anhydride de 3,4-dicarboxyphényle, 5-(N-phtalimidoyl)pentyle, phénoxyméthyle et butyle tertiaire.

**2.** Composition selon la revendication 1, dans laquelle R est $(CH_2)_3$.

**3.** Composition selon la revendication 2, dans laquelle $R^1$ est un groupe phényle éventuellement substitué.

**4.** Composition selon la revendication 3, dans laquelle $R^1$ est choisi parmi un groupe phényle, 4-méthylphényle, 4-nitrophényle, 4-(tert-butyl)phényle, 4-chlorophényle et 4-méthoxyphényle.

**5.** Composition selon la revendication 2, dans laquelle $R^1$ est pentyle, octyle ou phénoxyméthyle.

**6.** Composition selon la revendication 1, dans laquelle R est $(CH_2)_5$.

**7.** Composition selon la revendication 1, dans laquelle R est alkylène en $C_6$.

**8.** Composition selon la revendication 7, dans laquelle $R^1$ est un groupe phényle éventuellement substitué.

**9.** Composition selon l'une quelconque des revendications précédentes, qui est un détergent pour laver le linge.

**10.** Composition de blanchiment selon l'une quelconque des revendications précédentes, qui comprend au moins un autre activateur de blanchiment en mélange avec ledit composé hétérocyclique N-acylé.

**11.** Composition selon la revendication 10, dans laquelle l'autre activateur de blanchiment comprend une éthylènediamine tétraacylée, de préférence la tétraacétyléthylènediamine.

**12.** Composition de blanchiment selon la revendication 10, dans laquelle l'autre activateur de blanchiment comprend le nonanoyloxybenzènesulfonate de sodium.

**13.** Procédé dans lequel on fait réagir une source de peroxygène avec un composé activateur tel que défini dans l'une quelconque des revendications 1 à 8 dans une étape de perhydrolyse dans une solution aqueuse afin de former

une solution de produit contenant un produit oxydé et on utilise la solution de produit en tant qu'agent de blanchiment.